Europäisches Patentamt

European Patent Office

Office européen des brevets

(11) Publication number: **0 268 376**
**A2**

# EUROPEAN PATENT APPLICATION

(21) Application number: **87309158.1**

(22) Date of filing: **16.10.87**

(51) Int. Cl.⁴: **G01N 33/72**

(30) Priority: **22.10.86 US 921519**

(43) Date of publication of application:
**25.05.88 Bulletin 88/21**

(84) Designated Contracting States:
**DE FR GB IT**

(71) Applicant: **HELENA LABORATORIES
CORPORATION
1530 Lindbergh Drive
Beaumont Texas 77704(US)**

(72) Inventor: **Labbe, Robert F.
University of Workington School of Medicine
Seattle Washington 98195(US)**
Inventor: **Rettmer, Rebecca L.
University of Washington School of
Medicine
Seattle Washington 98195(US)**

(74) Representative: **Gore, Peter Manson et al
W.P. THOMPSON & CO. Coopers Building
Church Street
Liverpool L1 3AB(GB)**

(54) **Blood sample modification and reagent compositions therefor.**

(57) A method of forming a modified blood sample or compound which comprises reacting a blood sample with a reagent which forms a modified blood sample or compound which has substantially the same spectral properties as oxygenated hemoglobin or a non-deteriorated blood sample.

EP 0 268 376 A2

## BLOOD SAMPLE MODIFICATION AND REAGENT COMPOSITIONS THEREFOR

The present invention relates to the formation of modified blood samples or compounds. More particularly it relates to the assay of zinc protoporphyrin in whole blood and especially to a reagent composition that permits the determination of zinc protoporphyrin levels by hematofluorometry without the required oxygenation or re-oxygenation of hemoglobin in intact erythrocytes.

It has been well established that the determination of zinc protoporphyrin is important in the diagnosis and treatment of various diseases and deficiencies. For example, the determination of zinc protoporphyrin in blood is important in detecting iron deficiency, which effects an estimated 20% of the world's population, and in screening lead poisoning, which remains an environmental and occupational hazard. Also, the determination of zinc protoporphyrin is important and useful in monitoring therapy or treatment for either of the above noted conditions.

For more than 10 years, a common method to screen for chronic lead exposure or iron deficiency has been to measure the zinc protoporphyrin level of whole blood. This has been most often accomplished using an instrument, the hematofluorometer, which is designed for front-surface fluorometry of whole blood. In making measurements, however, if the hemoglobin is not fully oxygenated, the hematofluorometer gives falsely low values due to a special shift of the hemoglobin. To overcome this problem, it was necessary in the past to verify that the hemoglobin was fully oxygenated, and this was accomplished by stirring or aeration of the blood specimens followed by three or more successive readings with the fluorometer. If the several readings were substantially identical, this was an indication of fully oxygenated hemoglobin and thus a relatively accurate zinc protoporphyrin level.

As may be appreciated, there are inherent possibilities for error in the aforementioned system. In addition, this approach to oxygenation is time consuming and thus, less economical. Finally, stored or aged blood becomes much more difficult, if not impossible, to fully oxygenate by existing procedures.

According to the present invention there is provided a method of forming a modified blood sample or compound which comprises reacting a blood sample with a reagent which forms a modified blood sample or compound which has substantially the same spectral properties as oxygenated hemoglobin or a non-deteriorated blood sample.

In accordance with one embodiment of the present invention there is provided a method of analyzing a blood sample, which is subject to deterioration, which comprises derivitizing a blood sample to form a blood compound having substantially the same properties as a non-deteriorated blood sample and conducting the analysis by fluorometry or the like.

A further embodiment of the present invention provides a method of analyzing a blood sample of zinc protoporphyrin which comprises subjecting the sample to hematofluorometry wherein a reagent is added to the blood sample to provide a modified blood sample having substantially the same spectral properties as oxygenated hemoglobin.

The present invention particularly provides a reagent composition for use in hematofluorometry or the like which comprises a source of cyanide ions and a stabilizing agent.

Prior to the present invention, there was no approach to making one, accurate and dependable reading of zinc protoporphyrin fluorescence by hematofluorometry. The present invention overcomes this shortcoming by providing a new and improved approach to zinc protoporphyrin analysis by reacting the hemoglobin with a reagent to produce a stable coordination product which has a similar spectrum (in the desired optical range) to oxygenated hemoglobin. More particularly, the present invention produces a derivitized hemoglobin which has a similar spectrum as oxygenated hemoglobin, or oxyhemoglobin the form of hemoglobin present when zinc protoporphyrin should actually be measured. To be effective for use in hematofluorometry, a derivitizing reaction must occur rapidly while preserving the erythocyte intact. The present invention includes a reagent and a method of use of the reagent to form a derivatized hemoglobin having the same or substantially the same spectral properties as oxyhemoglobin (within the spectral range under consideration utilizing a hematofluorometer).

The present invention provides this derivatized hemoglobin preferably through the use of a cyanide-based reagent composition. The use of the cyanide-based reagent composition results in a cyanide-hemoglobin derivative having the same or substantially the same spectral properties as hemoglobin. Whilst the present invention is particularly described with reference to the use of potassium cyanide, not only can other sources of cyanide be used but also other possible coordinating substances can be used to derivitize the hemoglobin.

The preferred reagent composition includes a source of cyanide, such as, for example, about 0.2 mol/L potassium cyanide as the active ingredient to form a cyanide hemoglobin coordination complex. The reagent composition should most desirably also include beta-D-fructofuranosyl-alpha-D-glucopyranoside (i.e., sucrose) in the amount of preferably about 0.3 mol/L as a stabilizer to provide better overall reaction conditions. However stabilizing agents other than beta-D-fructofuranosyl-alpha-D-glucopyranoside may be employed.

One critical factor is the cyanide ion concentration in the reagent, as it is this factor which affects the overall efficiency of the system. A second critical factor is the pH, which optimizes the reaction condition for rapid, quantitive conversion of the hemoglobin. Preferably potassium cyanide is the source of cyanide ions and effects pH control.

Using the ingredients and amounts referred to above, a total volume of 5L should be formulated. The concentration range of potassium cyanide should be 0.05-0.30 mol/l while the range of beta-D-fructo-furanosyl-alpha-D-flucopyranoside whould be 0.2-0.4 mol/l. All compounds should be ACS reagent grade. Preferably the molar ratio of cyanide to stabilizer is substantially 2:3. The pH of the system should be approximately 11 ± 0.2 to effect virtually instantaneous and complete reaction between cyanide and hemoglobin.

A particular manufacturing procedure is to first add beta-D-fructofuranosyl-alpha-D-glucopyranoside and stir until dissolved and then add the potassium cyanide and stir until dissolved.

In the use of the reagent composition of the present invention, one or two drops of the reagent composition is usually added to a typical, standard-sized sample (usually one or two drops) of whole blood heretofore used for fluorometric analysis.

## Claims

1. A method of forming a modified blood sample or compound which comprises reacting a blood sample with a reagent which forms a modified blood sample or compound which has substantially the same spectral properties as oxygenated hemoglobin or a non-deteriorated blood sample.

2. A method of analyzing a blood sample, which is subjected to deterioration, which comprises derivitizing the blood sample to form a blood compound having substantially the same properties as a non-deteriorated blood sample and conducting the analyzis by fluorometry or the like.

3. A method of analyzing a blood sample for zinc protoporphyrin which comprises subjecting the sample to hematofluorometry, wherein a reagent is added to the blood sample to provide a modified blood sample having substantially the same spectral properties as oxygenated hemoglobin.

4. A method according to any of claims 1 to 3, wherein the reagent or the meas of derivitizing comprises cyanide.

5. A method according to any of claims 1 to 4, wherein a stabilizer is also employed.

6. A reagent composition for use in hematoflurormetry or the like which comprises a source of cyanide ions and a stabilizing agent.

7. A reagent composition according to claim 6, wherein the source of cyanide ions is potassium cyanide to effect pH control.

8. A reagent composition according to claim 6 or 7 wherein the molar ratio of cyanide to stabilizer is substantially 2:3.

9. A reagent composition according to any of claims 6 to 8, wherein the stabilizer is beta-D-fructofuranosylalpha-D-glucopyranoside.